# EUROPÄISCHE PATENTSCHRIFT

(11) **EP 2 151 214 B1**
(45) Veröffentlichungstag und Bekanntmachung des Hinweises auf die Patenterteilung: **23.01.2013**
(21) Anmeldenummer: 08161505.6
(22) Anmeldetag: 30.07.2008
(51) Int. Cl.: A61C 13/00, A61K 6/00

(54) **Lichthärtende Schlicker für die stereolithographische Herstellung von Dentalkeramiken**
Light hardening dross for stereolithographic production of dental ceramics
Barbotine durcissant à la lumière pour la fabrication stéréolithographique de céramiques dentaires

(43) Veröffentlichungstag der Anmeldung: 10.02.2010
(73) Patentinhaber: Ivoclar Vivadent AG, 9494 Schaan (LI); Technische Universität Wien, 1040 Wien (AT)
(72) Erfinder: Moszner, Norbert, 9493 Mauren (LI); Wachter, Wolfgang, 9494 Schaan (LI); Appert, Christoph, 9470 Vaduz (LI); Rheinberger, Volker, 9490 Vaduz (LI); Liska, Robert, 2123 Schleinbach (AT); Stampfl, Jürgen, 1050 Wien (AT); Patzer, Johannes, 1110 Wien (AT)
(74) Vertreter: UEXKÜLL & STOLBERG

(56) Entgegenhaltungen:
- EP-A- 1 716 836
- DE-A1-102005 058 116
- US-A- 5 496 682
- US-A- 6 117 612

## Beschreibung

Die vorliegende Erfindung betrifft lichthärtende Schlicker für die stereolithographische Herstellung von Keramikformteilen, wie z.B. dentalen Inlays, Onlays, Veneers, Kronen, Brücken und Gerüsten.

Unter dem Begriff "Rapid Prototyping" (RP), werden generative Fertigungsverfahren zusammengefasst, bei denen aus computergestützten Konstruktionsdaten (CAD-Daten) 3-dimensionale Modelle oder Bauteile hergestellt werden (A. Gebhardt, Vision of Rapid Prototyping, Ber. DGK 83 (2006) 7-12). Dabei handelt es sich um Verfahren, wie z.B. Stereolithographie (SL), selektives Lasersintern (SLS), 3-D-Printing, Fused Deposition Modelling (FDM), Ink-Jet-Printing (IJP), 3D-Plotting, Multi-Jet Modelling (MJM), Solid Freeform Fabrication (SFF), Laminated Object Manufacturing (LOM), Laser Powder Forming (LPF) und Direct Ceramic Jet Printing (DCJP), mit denen sich Modelle, Bau- oder Formteile auch in Kleinserie kostengünstig herstellen lassen (A. Gebhardt, Generative Fertigungsverfahren, 3. Aufl., Carl Hanser Verlag, München 2007, 77ff.). Bei der Stereolithographie handelt es sich um RP-Verfahren (A. Beil, Fertigung von Mikro-Bauteilen mittels Stereolithographie, Düsseldorf 2002, VDI-Verlag 3 ff.), bei denen ein Formteil auf der Basis von CAD-Daten schichtweise aus einem flüssigen und härtbaren Monomerharz aufgebaut wird.

Stereolithographische Verfahren zur Herstellung von dentalen Formkörpern, wie Inlays, Kronen oder Brücken, sind vor allem bei keramischen Werkstoffen von grossem Vorteil, da damit eine deutliche Vereinfachung der im zahntechnischen Labor mit grossem manuellen Aufwand betriebenen Abform- und Giessprozesse bzw. der Fräs- und Schleifoperationen möglich ist und gleichzeitig der bei nicht-generativen Verfahren auftretende grosse Materialverlust vermieden werden kann. Da heutzutage eine vollständige digitale Prozesskette etabliert ist, lassen sich die klassischen Verfahrensschritte zur Herstellung von z.B. mehrgliedrigen Brückengerüsten (Ausrichten im Artikulator, Wachsmodulation, Einbetten und Guss) durch die Digitalisierung des Modells, virtuelle Konstruktion des dentalen Formkörpers und dessen generative stereolithographische Fertigung ersetzen.

Bei der stereolithographischen Herstellung von keramischen Formteilen wird zunächst ein keramischer Grünling durch schichtweise Strahlungshärtung eines fliessfähigen keramischen Schlickers hergestellt, der dann nach der Entbinderung zu einem dichten keramischen Formkörper gesintert wird. Der Grünling wird auch Grünkörper genannt. Als Entbinderung wird das Austreiben des Bindemittels bezeichnet. Hierbei wird das verwendete Bindemittel üblicherweise durch Erhitzen des Grünlings auf eine Temperatur von ca. 90 °C bis 600 °C entfernt. Wesentlich ist, dass die Bildung von Rissen und Deformationen weitestgehend vermieden wird. Durch die Entbinderung wird aus dem Grünling der sogenannte Weissling.

Beim Entbindern finden rein thermische wie auch thermochemische Prozesse statt. Üblicherweise werden beim Verpressen von keramischen Pulvern als Binder Mischungen von Wasser, Lösungsmitteln, Polymeren, Wachsen oder Ölen eingesetzt. Als Polymere finden meist Polypropylen, Polyethylen, Polyvinylacetat, Polyvinylalkohol, Methylcellulose, Polyvinylpyrrolidon, Polystyrol oder Polyethylmethacrylat Anwendung (vgl. R. Moreno, Amer. Cer. Soc. Bull. 71 (1992) 1647-1657). Hierbei handelt es sich um lineare Polymere, die durch Depolymerisation oder Kettenspaltung bei höherer Temperatur mehr oder weniger leicht zu flüchtigen Komponenten abgebaut werden.

Im Falle stereolithographisch erzeugter Grünlinge auf der Basis von vernetzenden Monomermischungen liegt ein Polymernetzwerk vor. Durch die Verwendung vernetzender Monomere kann die Härtungszeit, die erforderlich ist, um einen stabilen Festkörper zu erhalten, deutlich verkürzt werden, gleichzeitig weist das sich ausbildende Polymernetzwerk im Vergleich zu linearen Polymeren aber auch eine deutlich erhöhte thermische Stabilität auf, was den Entbinderungsprozess nachteilig beeinflusst.

Die Sinterung des Weisslings erfolgt im Sinterofen beim Hochtemperaturbrand. Dabei kommt es zur Verdichtung und Verfestigung des fein verteilten Keramikpulvers durch Temperatureinwirkung unterhalb der Schmelztemperatur der Hauptkomponenten, wodurch das poröse Bauteil kleiner wird und dessen Festigkeit zunimmt.

Die EP 1 021 997 A2 beschreibt die Anwendung des Laser-Sinterverfahrens für die Herstellung von Zahnrestaurationen. Hierbei werden Metallpulver schichtweise unter Einsatz eines Lasers gesintert.

Die DE 101 14 290 A1 betrifft die Herstellung von dentalen Formteilen durch 3-D-Plotting unter Verwendung von schmelzbaren, kondensierbaren, thermisch oder mit UV- oder sichtbarem Licht härtbaren, ungefüllten oder gefüllten Materialien. Zur Herstellung von Grünkörpern werden anorganische Pasten vorgeschlagen, die aus Glas-, Glaskeramik- oder Keramikpulver bestehen, das mit Lösungsmittel, Binder und Plastifikator in eine formbare Paste überführt wird. Die verwendeten Pulver sind nicht oberflächenmodifiziert.

Die WO 97/29901 beschreibt ein Verfahren und ein Gerät zur Herstellung 3-dimensionaler Teile aus einem flüssigen, härtbaren Medium. Dabei wird das Teil schichtenweise aufgebaut, indem jede einzelne Schicht mit einem Laser abgefahren und dabei ausgehärtet wird. Danach wird mittels eines Abstreifers die nächste Schicht des härtbaren Materials aufgetragen und anschliessend ebenso gehärtet.

Ein stereolithographisches Verfahren zur Herstellung von Dentalimplantaten ist aus der WO 95/28688 bekannt.

Die US 5,496,682 offenbart lichthärtbare Zusammensetzungen für die Herstellung dreidimensionaler Körper durch Stereolithographie, die 40 bis 70 Vol.-% Keramik- oder Metallpartikel, 10 bis 35 Gew.-% Monomer, 1 bis 10 Gew.-% Photoinitiator, 1 bis 10 Gew.-% Dispergiermittel und vorzugsweise auch Lösungsmittel, Weichmacher und Kopplungsmittel enthalten.

Die US 6,117,612 beschreibt Harze für die stereolithographische Herstellung von gesinterten Keramik- oder Metallteilen. Die Harze haben eine Viskosität von weniger als 3000 mPa·s. Zur ihrer Herstellung werden Monomere mit geringer Viskosität eingesetzt, vorzugsweise in wässriger Lösung. Durch die Verwendung von Dispersionsmitteln soll ein hoher Feststoffgehalt bei geringer Viskosität erzielt werden.

Die DE 10 2005 058 116 A1 offenbart Suspensionen zur stereolithographischen Herstellung von keramischen Implantaten auf die in der US 6,117,612 beschriebene Weise, die keine Verdünnungsmittel wie Wasser oder organische Lösungsmittel enthalten, da diese durch lokales Verdampfen beim Energieeintrag die Viskosität erhöhen sollen. Die Viskosität der Suspension wird durch Variation der Konzentration eines Dispergators auf weniger als 20 Pa.s eingestellt. Als Dispergator werden Alkylammoniumsalze von Copolymeren mit sauren Gruppen eingesetzt, wobei diese auch auf die Partikel des keramischen Pulvers geschichtet werden können.

In der US 2005/0090575 A1 werden Methoden und Zusammensetzungen für die stereolithographische Herstellung von keramischen Bauteilen beschrieben. Es wird angegeben, dass mit den aus US 5,496,682 bekannten flüssigen Materialien hergestellte Formteile weich sind und daher einen zusätzlichen Härtungsschritt erfordern, um Verformungen beim Brennen zu vermeiden, während aus pastenförmigen Materialien gewonnenen Formkörper bei der Entbinderung innere Spannungen aufbauen, die beim Sintern zu Rissen führen. Zur Vermeidung dieser Probleme werden Weichmacher eingesetzt und die Menge des Keramikpulvers so gewählt, dass die Viskosität der Zusammensetzungen mindestens 10.000 Pa.s beträgt.

In den DE 199 38 463 A1 und DE 199 50 284 A1 werden mit sichtbarem Licht härtbare Zusammensetzungen und deren Verwendung zur Herstellung von Dentalrestaurationen aus Kunststoffmaterialen mit RP-Verfahren beschrieben.

Bei RP-Verfahren kommt der Zusammensetzung und den Eigenschaften des strahlungshärtbaren Schlickers eine entscheidende Bedeutung zu. So ist insbesondere für eine hohe Dichte und Endfestigkeit sowie gute Passgenauigkeit des keramischen Formteils ein möglichst hoher Volumenanteil der keramischen Partikel im Schlicker notwendig. Weiterhin ist eine gut eingestellte Rheologie des Schlickers eine grundlegende Voraussetzung für den stereolithographischen Aufbau eines defektfreien Grünkörpers, wobei die Viskosität und das Fliessverhalten unter anderem von der Grösse und dem Gehalt der Keramikpartikel im Schlickes abhängen. Außerdem ist zur berücksichtigen, dass sich das Bindemittel bei der Entbinderung des Grünlings ohne Riss- und Spannungsbildung rückstandsfrei entfernen lässt.

Weiterhin ist von Bedeutung, dass die zur Herstellung der Grünlinge verwendeten Schlicker über einen ausreichenden Zeitraum lagerstabil sind und sich gegenüber dem Wannenmaterial, der Unterlagefläche und den übrigen Komponenten, mit denen er im Verlauf des Stereolithographieprozesses in Kontakt kommt, weitgehend innert verhalten.

Ein besondere Problem bei der Herstellung von Keramikformteilen durch RP-Verfahren ist die Farbgebung der Keramik, da die verwendeten Färbemittel den Entbinderungs- und Sinterprozess überstehen müssen.

Die bekannten Schlicker sind hinsichtlich der oben genannten Anforderungen nicht zufriedenstellend. Der Erfindung liegt daher die Aufgabe zugrunde, verbesserte lichthärtende Schlicker zur stereolithographischen Herstellung von Keramik- und Glaskeramikformteilen bereitzustellen, die den obigen Anforderungen genügen. Die Schlicker sollen Grünlinge mit ausreichender Festigkeit ergeben, die sich ohne Verformung, Riss- oder Spannungsbildung entbindern lassen und die beim Sintern hochfeste Keramiken ergeben, die für dentale Zwecke geeignet sind. Gemäß einer bevorzugten Ausführungsform sollen die Keramiken eine dem gewünschten Einsatzzweck angepasste Farbgebung aufweisen.

Erfindungsgemäß wird diese Aufgabe durch Schlicker auf der Basis von radikalisch polymerisierbarem Bindemittel, Polymerisationsinitiator und Füllstoff gelöst, die
(A) 5 - 65 Gew.-%, vorzugsweise 9 bis 65 Gew.-%, besonders bevorzugt 10 bis 40 Gew.-% polyreaktives Bindemittel,
(B) 0,001 - 1,0 Gew.-%, vorzugsweise 0,01 bis 1,0 Gew.-%, besonders bevorzugt 0,1 bis 1,0 Gew.-% Photoinitiator und
(C) 35 - 90 Gew.-%, vorzugsweise 50 bis 90 Gew.-%, besonders bevorzugt 60 bis 90 Gew.-% oberflächenmodifizierte Keramik- und/oder Glaskeramikpartikel,
jeweils bezogen auf die Gesamtmasse des Schlickers, un Lösungs-mittel enthalten.

Als polyreaktives Bindemittel A lassen sich besonders Polymerisations- und. Polyadditionsharze verwenden, die in der Regel aus einer Mischung von niedermolekularen oder oligomeren Monomeren bestehen, die eine oder mehrere polyreaktionsfähige Gruppen enthalten.

Im Falle von Polymerisationsharzen sind radikalisch und kationisch polymerisierbare Harze sowie Monomere für die ring-öffnende Metathesepolymerisation bevorzugt. Bei den Polyadditionsharzen sind vor allem Thiol-En-Harze und Michael-Reaktionsharze geeignet.

Als radikalische Polymerisationsharze lassen sich insbesondere mono- oder multifunktionelle (Meth)acrylate oder deren Mischung einsetzen. Bevorzugte Monomere sind Acrylate wie Hydroxyethyl-, Hydroxypropyl-, Benzyl-, Tetrahydrofurfuryl- oder Isobornylacrylat, sowie Di-, Tri- oder Tetraethylenglycoldiacrylat, Acrylatgruppen-terminierte Poly(ethylenglycol)e und Poly(propylenglycol)e, Hexandioldiacrylat, Trimethylolpropantriacrylat und Pentaerythrittetraacrylat. Dabei werden die di- oder multifunktionellen Acrylate in der organischen Schlickerphase vorzugsweise in Mischung mit Monoacrylaten eingesetzt.

Erfindungsgemäß sind Acrylate aufgrund ihrer im Vergleich zu den entsprechenden Methacrylaten höheren Reaktivität bevorzugt, was sich in einer schnelleren Aushärtung bemerkbar macht. Da organische Komponenten beim Entbindern entfernt werden, ist die Verwendung von Acrylaten aus toxikologischer Sicht unbedenklich, so dass die Reaktivitätsvorteile der Acrylate genutzt werden können.

Weiterhin bevorzugte Monomere sind Acrylamide wie N-Ethylacrylamid, N,N-Dimethylacrylamid, N-(2-Hydroxyethyl)acrylamid, N,N'-Diethyl-1,3-bis(acrylamido)-propan und 1,4-Bis(acryl-amido)-butan. Dabei werden die Bisacrylamide im Vergleich zu den Monoacrylamiden im organischen Bindemittel vorzugsweise im Überschuss eingesetzt. Der Anteil an Monoacrylamiden in der Komponente (A) beträgt bevorzugt 30 Gew.-% oder weniger.

Die Eigenschaften der Schlicker vor und nach der Lichthärtung lassen sich durch eine gezielte Kombinationen der Monomeren beeinflussen. Mischungen aus monofunktionellen und difunktionellen Monomeren zeichnen sich durch eine relativ geringe Viskosität und Reaktivität der Harzmischung aus, wobei Viskosität und Reaktivität mit dem Gehalt an monofunktionellen Monomeren abnehmen. Ein Gehalt an monofunktionellen Monomeren gewährleistet eine geringere Sprödigkeit und ein schnellere Entbinderung der durch Lichthärtung der Schlicker erhaltenen Grünlinge. Mischungen aus difunktionellen und trifunktionellen Monomeren weisen eine höhere Viskosität und Reaktivität auf, wobei Viskosität und Reaktivität mit dem Gehalt an trifunktionellen Monomeren zunehmen. Der Gehalt an trifunktionellen Monomeren bewirkt eine höhere Sprödigkeit und langsamere Entbinderung der Grünlinge. Reaktivität und Viskosität der Harzmischung sowie der Polymerisationsschrumpf werden weiterhin durch die Molmasse der Monomeren bestimmt, wobei mit zunehmender Molmasse die Reaktivität und Polymerisationsschrumpf abnehmen, während die Viskosität ansteigt. Schliesslich kann über die Polarität der Monomeren die Wechselwirkung mit dem Werkstoff der stereolithographischen Wanne, wie z.B. die Quellung des Materials der Polymerisationswanne, beeinflusst werden. Als Wannenmaterialien werden häufig Siliconelastomere verwendet. Durch die Verwendung von OH-gruppenhaltige Monomeren kann eine Quellung von Siliconelastomeren weitgehend vermieden werden.

Als kationische Polymerisationsharze lassen sich kationisch ringöffnend polymerisierbare Monomere wie z.B. Glycidylether oder cycloaliphatische Epoxide, cyclische Ketenacetale, Spiroorthocarbonate, Oxetane oder bicyclische Orthoester einsetzen. Bevorzugte Beispiele sind: 2-Methylen-1,4,6-trioxaspiro[2.2]-nonan, 3,9-Dimethylen-1,5,7,11-tetraoxaspiro[5.5]undecan, 2-Methylen-1,3-dioxepan, 2-Phenyl-4-methylen-1,3-dioxolan, Bisphenol-A-diglycidylether, 3,4-Epoxy-cyclohexylmethyl-3,4-epoxycyclohexancarboxylat, Bis(3,4-epoxycyclohexylmethyl)adipat, Vinylcyclohexendioxid, 3-Ethyl-3-hy-droxymethyloxetan, 1,10-Decandiyl-bis-(oxymethylen)-bis-(3-ethyloxetan) oder 3,3-(4-Xylylendioxy)-bis-(methyl-3-ethyl-oxetan) sowie die in der EP 0 879 257 B1 genannten Epoxide. Bevorzugte Monomere sind Bisphenol-A-diglycidylether, 3,4-Epoxy-cyclohexylmethyl-3,4-epoxycyclohexancarboxylat und Bis(3,4-epoxycyclohexylmethyl)adipat.

Als kationisch polymerisierbare Matrixsysteme eignen sich auch Kieselsäurepolykondensate, die kationisch polymerisierbare Gruppen, bevorzugt Epoxid-, Oxetan-, oder Spiroorthoestergruppen tragen, und die beispielsweise durch hydrolytische Kondensation von Silanen zugänglich sind. Solche Kieselsäurepolykondensate sind beispielsweise in der DE 41 33 494 C2 oder US 6,096,903 beschrieben. Bevorzugte Kieselsäurepolykondensate sind solche, die durch hydrolytische Homo- oder Cokondensation von 2-(3,4-Epoxycyclohexyl-trimethoxysilan- und/oder -triethoxysilan erhalten werden. Weiterhin lassen sich als kationisch polymerisierbare Monomere auch Vinylether, wie z.B. Ethyl- oder Isobutylvinylether, sowie N-Vinylpyrrolidon einsetzen.

Außerdem können auch Mischungen von radikalisch und kationisch polymerisierbaren Monomeren eingesetzt werden.

Als Monomere für die ringöffnende Metathesepolymerisation (RÖMP) lassen sich bekannte RÖMP-Monomere, wie monocyclische Alkene oder Alkadiene, beispielsweise Cyclopenten, Cyclohepten, Cycloocten, Cyclododecen oder 1,5-Cyclooctadien, oder bicyclische Alkene, beispielsweise Bicyclo[2.2.1]hept-2-en (2-Norbornen) oder davon abgeleitete Derivate, wie 7-Oxa-bicyclo[2.2.1]hept-2-en, Bicyclo[2.2.1]hept-5-en-2,3-dicarbon-säuredimethylester, 7-Oxabicyclo[2.2.1]hept-5-en-2,3-dicarbon-säurediethylester, 5-Norbornen-2-methylester oder 5-Norbornen-2-yl-ester von Mono-, Di- und Multicarbonsäuren oder die Umsetzungsprodukte von 5-Norbornen-2-methanol oder 5-Norbornen-2-ol mit Mono- oder Diisocyanaten einsetzen. Dabei lassen sich die RÖMP-Monomere z.B. auch in Mischung mit radikalisch polymerisierbaren Monomeren, insbesondere mit mono- oder multifunktionellen (Meth)acrylaten verwenden.

Als polyreaktives Bindemittel A eignen sich insbesondere auch Thiol-En-Harze, die aus Mischungen von mono- oder multifunktionellen Mercaptoverbindungen und di- oder multifunktionellen ungesättigten Monomeren, vor allem Allyl- oder Norbonenverbindungen bestehen. Beispiele für mono- oder multifunktionellen Mercaptoverbindungen sind o-, m- oder p-Dimercaptobenzol und Ester der Thioglykol- oder der 3-Mercaptopropionsäure von Ethylen-, Propylen- oder Butylenglykol, Hexandiol, Glycerin, Trimethylolpropan oder Pentaerythrit. Beispiele für di- oder multifunktionelle Allylverbindungen sind Ester von Allylalkohol mit Di- oder Tricarbonsäuren, wie Malon-, Malein- Glutar-, Bernstein-, Adipin-, Sebacin-, Phthal-, Terephthal- oder Gallussäure sowie mono- oder trifunktionelle Allylether, wie z.B. Diallylether, α,ω-Bis[allyloxy]alkane, Resorcin- oder Hydrochinondiallylether sowie Pyrogalloltriallylether, oder andere ungesättigte Verbindungen wie z.B. 1,3,5-Triallyl-1,3,5-triazin-2,4,6-(1H,3H,5H)-trion, Tetraallylsilan oder Tetraallylorthosilikat. Beispiele für di- oder multifunktionelle Norbonenverbindungen sind Diels-Alder-Additionsprodukte von Cyclopentadien oder Furan mit di- oder multifunktionellen (Meth)acrylaten, sowie Ester bzw. Urethane von 5-Norbornen-2-methanol oder 5-Norbornen-2-ol mit Di- oder Polycarbonsäuren, wie z.B. Malon- Malein-, Glutar-, Bernstein-, Adipin-, Sebacin-, Phthal-, Terephthal- oder Gallussäure, mit bzw. Di-oder Polyisocyanaten, wie Hexamethylendiisocyanat bzw. dessen cyclisches Trimer, 2,2,4-Trimethylhexamethylendiisocyanat, Toluylendiisocyanat oder Isophorondiisocyanat. Dabei zeichnen sich Thiol-En-Harze vorteilhafter Weise durch eine geringe Viskosität und einen geringen Polymerisationsschrumpf aus. Darüber hinaus führen die Mercaptogruppen der Thiol-Komponente zu einer Wechselwirkung mit der Oberfläche der Keramikpartikel und stabilisieren somit den Schlicker.

Besonders bevorzugte Thiol-En-Komponenten sind Mischungen aus Estern der 3-Mercaptopropionsäure mit Hexandiol, Glycerin, Trimethylolpropan und/oder Pentaerythrit mit einer oder mehreren der folgenden En-Komponenten: Bicyclo[2.2.1]hept-2-en, Bicyclo[2.2.1]hept-5-en-2,3-dicarbonsäuredimethylester, 7-Oxa-bicyclo[2.2.1]hept-5-en-2,3-dicarbonsäurediethylester, 5-Norbornen-2-methylester der Bernsteinsäure oder Adipinsäure sowie Ester von Allylalkohol mit Adipin-, Terephthal- oder Gallussäure, Hydrochinondiallylether, Pyrogalloltriallylether und 1,3,5-Triallyl-1,3,5-triazin-2,4,6-(1H,3H,5H)-trion.

Als polyreaktives Bindemittel lassen sich auch sog. Michael-Reaktionsharze einsetzen. Dabei handelt es sich vorzugsweise um Mischungen von Acylamiden und/oder Bisacrylamiden, insbesondere di- und/oder multifunktionellen Acrylaten mit di- oder multifunktionellen Acetoacetaten. Beispiele für geeignete Acrylate sind Ethylenglycoldiacrylat, Hexandioldiacrylat, Tripropylenglycoldiacrylat, ethoxyliertes Bisphenol-A-Diacrylat, Polyethylenglycol-200-diacrylat, Trimethylolpropantriacrylat, Pentaerythrittetraacrylat. Diese Acrylate lassen sich insbesondere mit di-, tri- oder tetrafunktionellen Acetoacetaten, wie z.B. Hexandioldiacetoacetat, Trimethylolpropan- und Glycerintrisacetoacetat sowie Pentaerythrittetrakisacetoacetat zu Netzwerkpolymeren umsetzen. Dabei kann die Bildung geeigneter Katalysatoren und damit die Michael-Reaktion zwischen den di-oder multifunktionellen Acrylaten mit den di- oder multifunktionellen Acetoacetaten photochemisch induziert werden. Als Katalysatoren für die Michael-Reaktion sind starke Basen besonders geeignet, wie z.B. 1,5-Diazabicyclo[4.3.0]non-5-en (DBN), die durch Bestrahlen mit Licht aus entsprechenden Precursoren freigesetzt werden können. Die photoinduzierte Freisetzung von DBN aus N-benzylierten Precursoren ist z.B. in: K. Dietliker, T. Jung, K. Studer, J. Benkhoff, Chimia (2007) 655-660 beschrieben. Weitere sogenannte photolatente Basen werden unten beschrieben.

Werden die Acrylate im Überschuss zugesetzt oder enthalten die Acetoacetate polymerisationsfähige Gruppen, wie z.B. 2-Acetoacetoxyethylmethacrylat, oder werden die Acrylate in einem Mono- oder Dimethacrylat oder deren Mischungen gelöst, so kann simultan mit der Bildung eines Polyadditionsnetzwerkes auch ein radikalisches Polymernetzwerk gebildet werden. Durch die Struktur und Funktionalität der Acetoacetate und Acrylate lassen sich die Elastizität, Festigkeit der Polyadduktmatrix sowie deren Abbau im Entbinderungsprozess gezielt variieren. So sind difunktionelle Komponenten besonders bevorzugt, die im Vergleich zu tri- oder tetrafunktionellen Komponenten zu einer vergleichsweise geringen Vernetzungsdichte und deshalb besseren Entbinderbarkeit führen. Durch die Wahl von Spacergruppen kann die Flexibilität der Polymermatix beeinflusst werden. Unter Spacergruppen - auch Abstandshalter genannt - versteht man Reste, die zwei oder mehrere funktionelle Gruppen, wie z.B. Acetoacetat- oder Acrylat-Gruppen miteinander verbinden. Beispielsweise ergeben längere und/oder unpolare Spacergruppen, wie z.B. Decamethylen-Gruppen, im Vergleich zu Propylen- oder Methylenoxymethylen-Spacern eine höhere Flexibilität. Erfindungsgemäß werden vorzugsweise Mischungen verwendet, die weniger als 20 Gew.-% Monoacrylate und Monoacetoacetate enthalten.

Die Auswahl des Photoinitiators **B** richtet sich nach dem verwendeten Monomertyp. Schlicker auf der Basis von radikalisch polymerisierbaren Harzen sowie von Thiol-En-Polyadditionsharzen lassen sich mit den bekannten radikalischen Photoinitiatoren für den sichtbaren Bereich (vgl. J.P. Fouassier, J.F. Rabek (Hrsg.), Radiation Curing in Polymer Science and Technology, Vol. II, Elsevier Applied Science, London und New York 1993), wie z.B. Acyl- der Bisacylphosphinoxide, vorzugsweise mit α-Diketonen, wie 9,10-Phenanthrenchinon, Diacetyl, Furil, Anisil, 4,4'-Dichlorbenzil und 4,4'-Dialkoxybenzil, und besonders bevorzugt Campherchinon polymerisieren. Zur Beschleunigung der Initiierung werden α-Diketone vorzugsweise in Kombination mit aromatischen Aminen verwendet. Redoxsysteme, die sich besonders bewährt haben, sind Kombinationen aus Campherchinon mit Aminen, wie N,N-Dimethyl-p-toluidin, N,N-Dihydroxyethyl-p-toluidin, 4-Dimethylaminobenzoesäureethylester oder strukturverwandte Systeme.

Besonders bevorzugte Photoinitiatoren sind Norrish-Typ-I-Photoinitiatoren, vor allem Monoacyltrialkyl- oder Diacyldialkylgermanium-Verbindungen, wie z.B. Benzoyltrimethylgermanium, Dibenzoyldiethylgermanium oder Bis (4-methoxybenzoyl)diethylgermanium. Dabei lassen sich auch Mischungen der verschiedenen Photoinitiatoren einsetzen, wie z.B. Dibenzoyldiethylgermanium in Kombination mit Campherchinon und 4-Dimethylaminobenzoesäureethylester.

Zur Vermeidung der Polymerisation von Monomeranteilen, die sich im Material der stereolithographischen Wanne gelöst oder eingelagert haben, ist es von besonderem Vorteil, solche Photoinitiatoren und Beschleuniger einzusetzen, die sich durch eine geringe Löslichkeit im Wannenmaterial auszeichnen. Hierdurch wird verhindert, dass die Polymerisation zu einem Verbund zwischen der Wanne und der stereolithographisch erzeugten Schicht führt. Ein solcher Verbund hätte zur Folge, dass beim Hochfahren des Substratträgers zur Erzeugung den nächsten Schicht, entweder das Bauteil von Plattform abgerissen oder das Bauteil bzw. die Wanne beschädigt würden. Verwendet man einen Initiator, der sich nicht im Wannenmaterial löst, so kann dort keine Polymerisation stattfinden.

Kommerzielle erhältliche Wannen bestehen oft aus hydrophobem Siliconelastomer (Polysiloxan). In diesem Fall sind insbesondere polare oder hydrophile Photoinitiatoren und Beschleuniger von Vorteil. Die Polarität bzw. Hydrophilie der Photoinitiatoren und Beschleuniger lässt sich durch den Einbau von entsprechenden funktionellen Gruppen, wie z.B. OH- oder ionischen Gruppen oder polaren Resten, wie z.B. Di- oder Triethylenglycol-Resten, erhöhen.

Darüber hinaus sind auch oligomere oder polymere Photoinitiatoren und Beschleuniger von Vorteil, wie z.B. Oligomere oder Polymere des polymerisationsfähigen Photoinitiators 10-Methacryloyloxycampherchinon bzw. des polymerisationsfähigen Beschleunigers 2-(Dimethylamino)ethylmethacrylat, die aufgrund ihres hohen Molekulargewichts nur schwer in das Wannenmaterial eindringen können. Polymere Photoinitiatoren und Beschleuniger lassen sich auch dadurch erhalten, dass polymerisierbare Initiatoren oder Beschleuniger mit polaren Comonomeren, wie z.B. 2-Hydroxyethylmethacrylat, oder unpolaren Comonomeren, wie z.B. Styrol, thermisch copolymerisiert werden. Auf diese Weise lassen sich die Polarität und die Molmasse gezielt einstellen und damit die Löslichkeit der Photoinitiatoren und/oder Beschleuniger im Material der stereolithographischen Wanne oder anderen Materialien wirkungsvoll verringern.

Erfindungsgemäße Schlicker auf der Basis von kationisch polymerisierbaren Harzen lassen sich mit den bekannten kationischen Photoinitiatoren, besonders mit Diaryliodonium- oder Triarylsulfoniumsalzen, ggf. in Gegenwart geeigneter Sensibilisatoren, wie z.B. Campherchinon, Monoacyltrialkyl- oder Diacyldialkylgermanium-Verbindungen, wie z.B. Benzoyltrimethylgermanium, Dibenzoyldiethylgermanium oder Bis(4-methoxybenzoyl)diethylgermanium, aushärten. Beispiele für geeignete Diaryliodoniumsalze, die mit Campherchinon, Monoacyltrialkyl- oder Diacyldialkylgermanium-Verbindungen oder Thioxanthonen als Sensibilisator im sichtbaren Bereich eingesetzt werden können, sind die kommerziell zugänglichen 4-Octyloxy-phenylphenyl-iodoniumhexafluoroantimonat oder Isopropylphenyl-methylphenyl-iodoniumtetrakis(pentafluorophenyl)borat.

Für die Initiierung der mit sichtbarem Licht induzierten ringöffnenden Metathesepolymerisation von Schlickern auf der Basis von RÖMP-Mononeren eignen sich vor allem Rutheniumkomplexe, die einen N-heterocyclischen Carbenliganden tragen, vorzugsweise Rutheniumkomplexe gemäß der folgenden Formel, wobei R₁ und R₂ Alkylgruppen, vor allem Methyl, darstellen und R₂ auch Phenyl, Halogen oder Alkoxy sein kann:

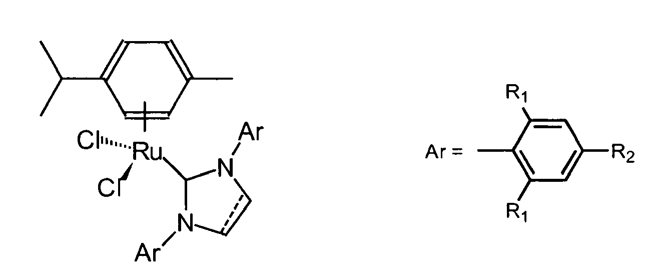

Zur Auslösung der lichtinduzierten Michael-Addition von Schlickern auf der Basis von Michael-Reaktionsharzen lassen sich sog. photolatente Basen einsetzen. Hierbei handelt es sich um Verbindungen, die beim Einstrahlen von Licht katalytisch wirksame tertiäre Aminkomponenten bilden. Beispiele hierfür sind photolatente Amidine, wie z.B. 5-Benzyl-1,5-diazabicyclo[4.3.0]non-5-en, das bei Bestrahlung 1,5-Di-azabicyclo[4.3.0]-5-nonen bildet, welches ein sehr wirksamer Katalysator für die Michael-Reaktion von Acetoacetaten mit Acrylaten ist. Photolatente Basen können auch mit bekannten Sensibilisatoren für den sichtbaren Bereich kombiniert werden. Als Photosensibilisatoren sind α-Diketone, wie 9,10-Phenanthrenchinon, Diacetyl oder besonders Campherchinon bevorzugt.

Als Komponente **C** enthalten die erfindungsgemäßen Schlicker oberflächenmodifizierte Keramik- und/oder Glaskeramikpartikel.

Unter Keramiken werden anorganische Werkstoffe verstanden, die eine kristalline Struktur aufweisen und meist aus entsprechenden Pulvern hergestellt werden. Vorzugsweise erfolgt die Herstellung der Keramik durch Sintern (Sinterkeramik). Oxidkeramiken werden vorzugsweise durch Sintern von Metalloxidpulvern wie z.B. ZrO₂ oder Al₂O₃ erhalten. Darüber hinaus können Oxidkeramiken auch eine oder mehrere Glasphasen enthalten. Bei Glaskeramiken handelt es sich um Werkstoffe, die meist aus amorphen Gläsern, insbesondere Silikatgläsern, durch gesteuerte Kristallisation hergestellt werden und bei denen eine Glasphase und eine oder mehrere Kristallphasen im Festkörper nebeneinander vorliegen. Bei sinterbaren Glaskeramiken kann sowohl von Glaspulvern als auch von Glaskeramikpulvern ausgegangen werden.

Bevorzugt sind sinterbare Glaskeramikpartikel auf Basis Leucit- oder Lithiumdisilikat-verstärkten Gläsern und/oder Keramikpartikel auf der Basis von ZrO₂ oder Al₂O₃, vorzugsweise reinem ZrO₂ oder reinem Al₂O₃, Partikel auf der Basis von mit HfO₂, CaO, Y₂O₃, CeO₂ und/oder MgO stabilisiertem ZrO₂, Partikel auf der Basis von anderen Metalloxiden sowie keramischen Kompositmaterialien, die aus mehren Oxiden hergestellt werden und somit aus unterschiedlichen kristalline Oxidphasen aufgebaut sind, vorzugsweise ZrO₂-Al₂O₃, insbesondere reinem ZrO₂-Al₂O₃ oder mit HfO₂, CaO, Y₂O₃, CeO₂ und/oder MgO stabilisiertem ZrO₂-Al₂O₃.

Der Begriff "rein" ist im Sinne von "chemisch rein" zu verstehen, d.h. eine ZrO₂- bzw. Al₂O₃-Keramik ist nur aus ZrO₂ bzw. Al₂O₃ aufgebaut. Stabilisierte Keramiken enthalten neben dem Basisoxid wie ZrO₂ oder Al₂O₃ ein Stabilisisierungsmittel, das vorzugsweise aus HfO₂ CaO, Y₂O₃, CeO₂ MgO und Mischungen davon ausgewählt ist. Das Stablilisierungsmittel wird vorzugsweise in einer Menge von 3 bis 5 Gew.-% eingesetzt, bezogen auf die Masse der stabilisierten Keramik. Hochfeste ZrO₂-Keramiken enthalten zur Stabilisierung der tetragonalen Kristallstruktur vorzugsweise 3 bis 5 Gew.-% Y₂O₃ (Yttriumoxid). Diese ZrO₂-Keramik wird als Y-TZP (Yttrium Stabilized Tetragonal Zirconium Dioxide Polycrystals) bezeichnet. Keramikpartikel, die nur Basisoxid und Stabilisierungsmittel enthalten, sind besonders bevorzugt.

Die Partikelgröße der Komponente B liegt vorzugsweise im Bereich von 50 nm bis 50 µm. Sie ist abhängig von der eingesetzten Keramik. Bei Al₂O₃ liegt die Größe der als Komponente B verwendeten Partikel vorzugsweise im Bereich von 50 bis 500 nm, besonders bevorzugt zwischen 75 und 200 nm; bei Glaskeramik im Bereich von 500 nm und 50 µm, ganz bevorzugt zwischen 1 und 10 µm; bei TZP-3Y Zirkondioxid im Bereich von 50 und 500 nm, ganz bevorzugt zwischen 50 und 350 nm. Die Partikelgröße wird dabei vorzugsweise so gewählt, dass sedimentationsstabile Schlicker erhalten werden. Bei den Partikelgrößen handelt es sich um die absoluten Ober- und Untergrenzen.

Weiterhin lassen sich auch Keramik- oder Glaskeramikpartikel mit einer Partikelgröße im Bereich von 10-200 nm als Nano- der Organosole, d.h. als Dispersion der Nanopartikel in einem Lösungsmittel, einem geeigneten Monomer der Komponente A oder einer Mischungen daraus einsetzen.

Die Partikel werden mit geeigneten Stoffen oberflächenmodifiziert. Zur Oberflächenmodifizierung werde vorzugsweise solche Verbindungen eingesetzt, die chemisch, d.h. durch ionische oder kovalente Bindungen an die Oberfläche der Keramik- oder Glaskeramikpartikel gebunden werden. Bevorzugt sind Verbindungen, die entweder Säuregruppen, vorzugsweise Carbonsäure-, Phosphonsäure-, Hydrogenphosphatgruppen oder saure Phosphorsäureestergruppen, oder Silylgruppen, vorzugsweise Alkoxysilylgruppen enthalten. Die Partikeloberfläche kann teilweise oder vorzugsweise vollständig mit dem Modifizierungsmittel bedeckt sein. Bei den erfindungsgemäß verwendeten Modifizierungsmitteln handelt es sich um monomere Verbindungen.

Dabei sind erfindungsgemäß solche Verbindungen besonders geeignet, die im Gegensatz zu den sogenannten Haftvermittlern oder Kopplungsreagentien nur mit der Partikeloberfläche reagierende Gruppen enthalten, aber keine polyreaktiven Gruppen wie radikalisch polymerisierbaren Gruppen, z.B. (Meth)acryl-, (Meth)acrylamid-, Vinyl-, Vinylether- oder Epoxidgruppen, die mit der Harzmatrix (A) eine kovalente Bindung eingehen. Solche Verbindungen werden hierin als nicht polymerisierbare Oberflächenmodifikatoren bezeichnet. Diese Verbindungen haben den Vorteil, dass ein stabiler Verbund zwischen der Keramikpartikeloberfläche und der Polymermatrix im ausgehärteten Grünling nicht zustande kommt, was die vollständige Entfernung der Polymeranteile im Entbinderungsprozess vereinfacht.

Als nicht polymersisierbare Oberflächenmodifikatoren eigen sich insbesondere lineare oder verzweigte Carbonsäuren, wie z.B. Ameisensäure, Essigsäure, Propionsäure, Octansäure, Iso-buttersäure, Isovaleriansäure, Pivalinsäure, saure Phosphorsäureester, wie z.B. Dimethyl-, Diethyl-, Dipropyl-, Dibutyl-, Dipentyl-, Dihexyl-, Dioctyl- oder Di(2-ethylhexyl)phosphat, oder Phosphonsäuren, z.B. wie Methyl-, Ethyl-, Propyl-, Butyl-Hexyl, Octyl- oder Phenylphosphonsäure. Als nicht polymerisierbare Oberflächenmodifikatoren geeignete Silane sind beispielsweise Propyltrimethoxysilan, Phenyltrimethoxysilan, Hexyltrimethoxysilan, Octyltrimethoxysilan, Trimethylchlorsilan, Trimethylbromsilan, Trimethylmethoxysilan oder Hexamethyldisilazan.

Gemäß einer bevorzugten Ausführungsform enthalten die erfindungsgemäßen Schlicker als Komponente **D** einen Inhibitor zur Verhinderung einer spontanen Polyreaktion als Stabilisator. Die Inhibitoren bzw. Stabilisatoren verbessern die Lagerstabilität der Schlicker und verhindern darüber hinaus eine unkontrollierte Polyreaktion in der stereolithographischen Wanne. Die Inhibitoren werden vorzugsweise in einer solchen Menge zugesetzt, dass die Schlicker über einen Zeitraum von ca. 2-3 Jahre lagerstabil sind. Besonders bevorzugt werden die Inhibitoren in einer Menge von 0,001 bis 1,0 Gew.-%, ganz besonders bevorzugt 0,001 bis 0,50 Gew.-% eingesetzt, jeweils bezogen auf die Gesamtmasse des Schlickers.

Für radikalische Reaktionsharze und Thiol-En-Harze werden als sog. aerobe Inhibitoren Phenole, wie Hydrochinonmonomethylether (MEHQ) oder 2,6-Di-tert.-butyl-4-methyl-phenol (BHT), eingesetzt, die nur in Gegenwart von Sauerstoff effektiv wirksam sind und vorzugsweise in einem Konzentrationsbereich von 200-2000 ppm verwendet werden. Geeignete anaerobe Inhibitoren sind Phenothiazin, 2,2,6,6-Tetramethyl-piperindin-1-oxyl-Radikal (TEMPO), Iod und Cupfer-(I)-iodid. Diese wirken schon in geringen Konzentrationen von vorzugsweise 10-50 ppm auch bei Abwesenheit von Sauerstoff. Eine Polymerisation findet erst dann statt, wenn diese Additive verbraucht sind. Dabei ist es von Vorteil, eine Mischung von aeroben und anaeroben Inhibitoren einzusetzen. Als Inhibitoren für die kationische Polymerisation lassen sich vorteilhaft basisch Alkali- und Erdalkalisalze gesättigter und ungesättigter Carbonsäure einsetzen, beispielsweise Natriummethylat, Calciumstearat oder Lithiumoleat. Dagegen lässt sich die Michael-Reaktion durch Säuren inhibieren, beispielsweise durch Essigsäure oder p-Toluolsulfonsäure.

Aerobe Inhibitoren werden vorzugsweise in einer Menge von 0,01 bis 0,50 Gew.-% und anaerobe Inhibitoren in einer Menge von 0,001 bis 0,02 Gew.-% eingesetzt, jeweils bezogen auf die Gesamtmasse des Schlickers. Bevorzugte Mischungen enthalten 0,01-0,10 Gew.-% an aeroben Inhibitoren und 0,001- bis 0,01 Gew.-% an anaeroben Inhibitoren, ebenfalls bezogen auf die Gesamtmasse des Schlickers.

Gemäß einer weiteren bevorzugten Ausführungsform der Erfindung enthalten die Schlicker als Komponente E einen sogenannten Entbinderungsbeschleuniger. Dieser wird vorzugsweise in einer Menge von 0 bis 20 Gew.-%, besonders bevorzugt 0,01 bis 10 Gew.-% eingesetzt, jeweils bezogen auf die Gesamtmasse des Schlickers. Unter Entbindungsbeschleunigern werden Stoffe verstanden, welche das Entfernen des Bindemittels während des Entbinderungsprozesses erleichtern.

Die Entbinderung des Grünlings kann durch geeignete, d.h. polyreaktionswirksame Stoffe im Polyreaktionstiarz gefördert oder zielgerichtet beeinflusst werden. Dabei handelt es sich einerseits um Additive, die die Netzwerkbildung beeinflussen, wie vor allem kettenübertragungsaktive Stoffe, sog. Kettenregler, die zu einer Verringerung der Polymernetzwerkdichte und damit zu einer besseren thermischen Abbaubarkeit führen. Bekannte Kettenregler z.B. für die radikalische Polymerisation sind vor allem Mercaptane, wie z.B. Laurylmercaptan, und Disulfide. Disulfide, vor allem Dithiourethandisulfide, wie z.B. Tetramethylthiuramdisulfid oder Isopropylxanthogensäuredisulfid wirken bei der radikalischen Photopolymerisation als sog. Photoiniferter. Es handelt sich dabei um Verbindungen, die sowohl als Photoinitiator (Photoini-) wirken, als auch an Übertragungsreaktionen (engl.: transfer, -fer-) und Abbruchsreaktionen (engl.: termination, -ter) teilnehmen (vgl. vgl. T. Ostsu, M. Yoshida, Makromol. Chem., Rapid. Commun. 3 (1982) 127-132: Role of Initiator-Transfer Agent-Terminator (Iniferter) in Radical Polymerizations: Polymer Design by Organic Disulfides as Iniferters). Die Zugabe von kettenübertragungsaktiven Stoffen, d.h. von Kettenreglern oder Photoinifertern, bewirkt eine Verringerung der Netzwerkdichte des Polyreaktionsnetzwerkes, bei nahezu unveränderter Reaktivität der Polyreaktionsharzmischung. Kettenregler und Photoiniferter werden vorzugsweise in einer Menge von jeweils 0,005 bis 2 Gew.-% und besonders bevorzugt 0,01 bis 1,0 Gew.-% bezogen auf die Komponente (A) eingesetzt.

Als Entbinderungsbeschleuniger lassen sich erfindungsgemäß vorteilhaft auch Comonomere einsetzen, die zu einer Verringerung der thermische Stabilität von Polymernetzwerken führen. Hierzu eignen sich Comonomere, die thermisch labile Gruppen enthalten, wie z.B. Peroxid-, Azo- oder Urethangruppen, welche während des stereolithographischen Prozesses in das Polymernetzwerk eingebaut werden und dann im thermischen Entbinderungsprozess den Abbau des Polymernetzwerkes beschleunigen. Ein bevorzugtes Beispiel für ein polymerisationsfähiges Peroxid ist 4,4'-Divinylbenzoylperoxid, das durch Umsetzung von 4-Vinylbenzoylchlorid mit Natriumperoxid zugänglich ist. Ein bevorzugtes Beispiel für eine polymerisationsfähige AzoVerbindung ist der Ester aus 2-Hydroxyethylmethacrylat und der 4,4'-Azobis-(4-cyano-valeriansäure). Bevorzugte thermisch labile Urethane sind aus Diisocyanaten zugänglich, beispielsweise durch Umsetzung von 2,2,4-Trimethylhexamethylendiisocyanat (TMDI) oder Toluylendiisocyanat (TDI) mit Hydroxypropylacrylat (HPA) oder 2-Hydroxyethylacrylat (HEA). Ein weiteres Beispiel für einen thermisch labilen Monomerbaustein stellt α ,α,α',α'-Tetramethyl-1,4-benzen-dimethylacrylat dar, dessen Einbau in ein Michael-Addition-Netzwerke beispielsweise von Diacrylaten und Diacetoacetaten in Gegenwart katalytischer Säuremengen zu einem beschleunigten Abbau des Polymernetzwerks führt.

Außerdem sind als Entbinderungsbeschleuniger Comonomere geeignet, deren Polyreaktionsprodukte thermisch leicht abbaubar sind. Für radikalische Polymerisationsharze sind Comonomere bevorzugt, die wie α-Methylstyrol eine niedrige ceiling-Temperatur T_{c} aufweisen. Die ceiling-Temperatur ist die Grenztemperatur, bei der die Polymerisation mit der Depolymerisation im Gleichgewicht steht, und lässt sich aus dem Quotienten der Polymerisationsenthalpie und der Polymerisationsentropie berechnen (vgl. H.-G. Elias, Makromoleküle, Bd 1, 6. Aufl., Wiley-VCH, Weinheim etc. 1999, 193ff.). Beispielsweise beträgt T_{c} für α-Methylstyrol 61 °C. Die ceiling-Temperatur T_{C} von Polytetrahydrofuran (PTHF) liegt bei 80 °C. Dementsprechend kann z.B. durch Verwendung von Telechelen, insbesondere PTHF-Di(meth)acrylat-Telechelen als Comonomer die Abbaubarkeit von Poly(meth)acrylatnetzwerken beschleunigt werden. Erfindungsgemäß sind Comonomere mit einer ceiling-Temperatur von -10 bis 150 °C, vorzugsweise 10 bis 150 °C und besonders bevorzugt 20 bis 130 °C besonders geeignet. Die Comonomere werden vorzugsweise in einer Menge von 0,1 bis 30 Gew.-% und besonders bevorzugt 0,5 bis 20 Gew.% bezogen auf die Komponente (A) eingesetzt.

Die getrennte oder gemeinsame Verwendung der oben genannten Kettenregler, Photoiniferter und Comonomere zur Verbesserung der Entbinderung von Keramik- oder Glaskeramikgrünlingen ist ebenfalls ein Gegenstand der Erfindung.

Als Komponente **F** enthalten die erfindungsgemäßen Schlicker vorzugsweise eine farbgebende Komponente. Die farbgebende Komponente wird vorzugsweise in einer Menge von 0,00001 bis 2,0 Gew.-%, besonders bevorzugt 0,001 bis 1,0 Gew.-% und ganz besonders bevorzugt 0,01 bis 0,5 Gew.-% eingesetzt, bezogen auf die Masse der Komponente C.

Als farbgebende Komponente sind die üblichen Farbstoffe oder Pigmente erfindungsgemäß nicht geeignet, da sie nicht genügend stabil sind, um den Entbinderungs- oder Sinterungsprozess zu überstehen. Erfindungsgemäß werden als Komponente F reaktive Übergangsmetallverbindungen eingesetzt, die einerseits im Bindemittel A löslich sich und den Verlauf der Photohärtung nicht nachteilig beeinflussen und die andererseits während der Entbinderung des stereolithographisch hergestellten Keramikgrünlings oder der Sinterung des daraus erhaltenen Keramikweisslings farbgebende Übergangsmetallionen bilden. Als farbgebende Komponente bevorzugte Übergangsmetallverbindungen sind vor allem Acetylacetonate oder Carbonsäuresalze der Elemente Eisen, Cer, Praseodym, Terbium, Lanthan, Wolfram, Osmium, Terbium und Mangan. Bevorzugt sind die Salze der Carbonsäuren Essig-, Propion-, Butter-, 2-Ethylhexylcarbon-, Stearin- und Palmitinsäure. Besonders bevorzugt sind vor allem die entsprechenden Fe-, Pr-, Mn- und Tb-Verbindungen, wie z.B. Eisen(III)-acetat oder -acetylacetonat, Mangan(III)-acetat oder -acetylacetonat, Praseodym(III)-acetat oder -acetylacetonat oder Terbium(III)-acetat oder -acetylacetonat sowie die entsprechenden Carbonsäuresalze.

Vorzugsweise werden die farbgebenden Komponenten so gewählt, dass nach dem Entbindern und Sintern zahnfarbene Keramikformteile erhalten werden.

Die getrennte oder gemeinsame Verwendung der oben genannten Übergangsmetallverbindungen zur Einfärbung von Keramikformkörpern und besonders zur Einfärbung gesinterter Keramikformkörper, insbesondere die Verwendung als farbgebende Komponente bei RP-Verfahren zur Herstellung von Keramikformkörpern und besonders zur Herstellung gesinterter Keramikformkörper ist ebenfalls ein Gegenstand der Erfindung.

Erfindungsgemäß sind demgemäß solche Schlicker bevorzugt, die neben den Komponenten A, B und C einen Inhibitor D, einen Entbinderungsbeschleuniger E und/oder eine farbgebende Komponente F enthalten. Besonders bevorzugt sind Schlicker, welche die Komponenten A, B, C und D; A, B, C und E; A, B, C und F enthalten, ganz besonders bevorzugt sind Schlicker, welche die Komponenten A, B, C, D und E; A, B, C, D und F; A, B, C, E und F enthalten, und insbesondere Schlicker, welche die Komponenten A, B, C, D, E und F enthalten. Dabei ist es jeweils bevorzugt, die oben definierten Komponenten und bevorzugten Komponenten einzusetzen, vorzugsweise in den angegebenen Mengen.

Neben den Komponenten **A** bis **F** können die erfindungsgemäßen Schlicker weitere Komponenten als Additive enthalten.

Obwohl erfindungsgemäß auf die Anwendung von üblichen polymeren Dispergatoren zur Erreichung eines hohen Feststoffgehaltes verzichtet werden kann, da durch die Oberflächenmodifizierung mit den monomeren, nicht polymerisierbaren Oberflächenmodifikatoren die verdickende Wirkung der Keramikpartikel deutlich reduziert wird, können die Schlicker zusätzlich ein oder mehrere Dispergiermittel enthalten, die die Bildung von Agglomeraten und das Absetzen der Keramikpartikel weiter verhindern. Bevorzugte Dispergiermittel für wässrige Schlicker sind vor allem Polymere, insbesondere Polyelektrolyte, z.B. Polycarbonsäuren oder Polycarbonsäuresalze, oder nichtionische Polymere, wie z.B. Polyethylenglycol oder Carboxymethylcellulose. Als Dispergiermittel für wässrige Schlicker eignen sich Polyelektrolyte, die wie z.B. Ammoniumpolycarboxylat ionische Gruppen tragen und die deshalb relativ leicht an die Oberfläche von Festkörpern adsorbieren, z.B. an Keramikpartikel. Dabei können die Polyelektrolytionen den Partikeln eine elektrische Ladung verleihen, so dass man von einer elektrosterischen Wirkung spricht. Die dispergierende Wirkung der Polyelektrolyte hängt u.a. von ihrer Konzentration, der Art und dem Bedeckungsgrad der Partikel, dem pH-Wert der Lösung und deren Ionenstärke ab. Im Falle von organischen, nicht wässrigen Schlickern eignen sich als Dispergiermittel besonders Polymere, die im Polyreaktionsharz löslich sind, wie beispielsweise Poly(meth)acrylate, die in lichthärteten (Meth)acrylat-Harzen löslich sind. Dispergiermittel werden ggf. in einer Menge von vorzugsweise 0,5 bis 3 Gew.-% bezogen auf die Masse des Schlickers eingesetzt. Dispergiermittel mit einer Molmasse im Bereich von 500 bis 5.000 g/mol sind bevorzugt.

Die Partikel der Komponente C können schon vor der Schlickerherstellung mit Dispergiermittel vorbehandelt werden. Findet diese Behandlung vor der Oberflächenmodifizierung statt, werden die Partikel nur mit einer vergleichsweise geringen Menge an Dispergiermittel behandelt, vorzugsweise maximal 1 Gew.-%, um eine Behinderung der Oberflächenmodifizierung zu vermeiden. Werden die Partikel nach der Oberflächenmodifizierung mit Dispergiermittel behandelt, beträgt der Dispergiermittelanteil vorzugsweise 0,1 bis 5 Gew.-%, jeweils bezogen auf die Masse der Partikel.

Als weitere Komponenten können die erfindungsgemäßen Keramikschlicker einen oder mehreren Weichmacher enthalten. Der oder die Weichmacher können ggf. verhindern, dass der Keramikgrünling nach der photochemischen Aushärtung und einem evt. Trocknen brüchig wird. Darüber hinaus sorgen Weichmacher für eine eine ausreichende Flexibilität des stereolithographisch hergestellten Grünkörpers. Bevorzugte Weichmacher sind Phthalate, wie z.B. Dibutyl- oder Dihexylphthalat, nichtsaure Phosphate, wie z.B. Tributyl- oder Trikresylphosphat, n-Octanol, Glycerin oder Polyethylenglykole. Weichmacher werden vorzugsweise in einer Menge von 0 bis 15 Gew.-% und besonders bevorzugt 0,1 bis 5 Gew.-% eingesetzt, bezogen auf die Masse der Komponente (A).

Als Lösungsmittel eignen sich beispielsweise die oben als Weichmacher genannten Verbindungen.

Vorzugsweise werden als Lösungsmittel solche Komponenten eingesetzt, die eine Siedetemperatur von mindestens ca. 120 °C, vorzugsweise von 150 bis 250 °C, besonders bevorzugt von 180 bis 230 °C aufweisen, so dass es bei der stereolithographischen Verarbeitung des Schlickers nicht zu vorzeitigen Verdunstung kommt. Dabei sind Mischungen von solchen Lösungsmitteln besonders geeignet, die in einem Temperaturbereich zwischen 150 und 250. °C schrittweise verdampfbar sind. Ganz besonders geeignet sind Octanol, Triethylenglycol-Divinylether, 2-Amino-2-methyl-1-propanol, 2-Methyl-2,4-pentandiol, Ammoniumcitrat tribasisch (fest), Tripropylenglycol, Tetraethylenglycol, Triethylenglycol, Triethylcitrat, Acet-Essigsäure-Ethylester, Cyclohexanol, Cyclohexanon, Diethylenglycolmonomethylether, Oxalsäuredibutylester, 2.5-Dimethoxyterahydrofuran, Polythylenlycol 300, 1- oder 2-Nonanol, Diethylenglycoldiethylether, 2,5-Dimethoxytetrahydrofuran, Oxalsäuredibutylester, Cyclohexanol, Cyclohexanon, Acetessigsäureethylester und Mischungen davon.

Es wurde gefunden, dass das Verdampfen der obigen Lösungsmittel zur Bildung von Mikroporen im Grünling führt, die sich beim Sintern wieder schliessen, die aber das Entweichen der Gase im Entbinderungsschritt ermöglichen und fördern und so das Entstehen von Spannungen und Rissen verhindern. Außerdem wird die Gefahr einer Trennung der stereolithographisch erzeugten Schichten reduziert und eine vollständige Entfernung der organischen Komponenten begünstigt.

Das oder die Lösungsmittel werden vorzugsweise in einer Menge von 5 und 50 Gew.-%, besonders bevorzugt von 10 und 30 Gew.-% eingesetzt, bezogen auf die Masse der Komponente (A).

Alternativ kann eine Porosität des Grünlings auch dadurch erreicht werden, dass vor Wärmebehandlung herauswaschbare Anteile extraktiv entfernt werden. Geeignete extrahierbare Komponenten sind wasserlösliche Polymere, wie z.B. Polyvinylalkohol, Polyvinylpyrrolidon und Polyethylenglycole. Weiterhin können benzinlösliche Stoffe wie Parafine oder Wachse und langkettige Fettsäureester eingesetzt werden. Die bevorzugte Menge an extrahierbaren Komponenten in der Harzmatrix liegt zwischen 0 und 40 Gew.-%, besonders bevorzugt zwischen 0,1 und 30 Gew.-%, bezogen auf die Komponente (A).

Weiterhin können die erfindungsgemäßen Schlicker Komponenten enthalten, die beim Entbinderungsprozess den oxidativen Abbau der Polymermatrix fördern, wie z.B. bei Raumtemperatur stabile Peroxide, oder auch katalytisch wirksame Komponenten, die eine katalytische Entbinderung ermöglichen. Neben Peroxiden eignen sich auch andere Substanzen, die oxidierend wirken, wie z.B. Salpetersäure, oder die Oxidationsmittel abspalten oder bilden.

Außerdem können die erfindungsgemäß Schlicker Entschäumungsmittel und/oder Hautverhinderungsmittel enthalten, die die Schaumbildung bei der Herstellung der Schlicker bzw. die Bildung einer Haut während der Verarbeitung der Schlicker verhindern. Entschäumungs- und/oder Hautverhinderungsmittel werden vorzugsweise in eine Menge von jeweils 0 bis 5 Gew.-%, besonders bevorzugt 0,1 bis 2 Gew.-% in der organischen Matrix eingesetzt, bezogen auf die Masse der Komponente (A).

Die erfindungsgemäßen. Schlicker eignen sich besonders zur Herstellung von Keramik- oder Glaskeramikformteilen, insbesondere zur Herstellung von Dentalrestauration, wie z.B. Inlays, Onlays, Veneers, Kronen, Brücken oder Gerüsten.

Gegenstand der Erfindung ist weiterhin ein Verfahren zur Herstellung von Keramik- oder Glaskeramikformteilen bei dem man
(a) einen Grünling herstellt, indem man einen erfindunggemäßen Schlicker durch lokales Einbringen von Strahlungsenergie unter Ausbildung der geometrischen Form des Grünlings aushärtet,
(b) man dann den Grünling einer Wärmebehanldung zur Entfernung des Bindemittels (Entbinderung) unterwirft, um einen Weissling zu erhalten, und
(c) man den Weissling anschließend sintert.

Die Herstellung des Grünlings in Schritt (a) erfolgt durch Rapid Prototyping, vorzugsweise durch Stereolithographie. Es wird durch schichtweise Strahlungshärtung eines fliessfähigen keramischen Schlickers ein keramischer Grünling hergestellt, der in Schritt (b) entbindert wird. Hierbei wird das verwendete Bindemittel durch Erhitzen des Grünlings auf eine Temperatur von vorzugsweise 90 °C bis 600 °C entfernt, und es wird der sogenannte Weissling erhalten. Der Weissling wird in Schritt (c) zu einem dichten keramischen Formkörper gesintert. Die Sinterung des Weisslings erfolgt im Sinterofen, vorzugsweise bei einer Temperatur für Glaskeramik von 650 bis 1100 °C, bevorzugt 700 bis 900 °C, für Zirkondioxid von 1100 bis 1600 °C, bevorzugt 1400 bis 1500 °C und für Aluminiumoxid von 1400 bis 1800°C, bevorzugt 1600 bis 1700°C. Die nach dem erfindungsgemäßen Verfahren hergestellten Keramikformkörper zeichnen sich durch eine hohe Festigkeit und große Detailgenauigkeit aus. Die Biegefestigkeit nach ISO 6872 liegt bei Formkörpern aus Glaskeramik vorzugsweise über 100 MPa, insbesondere im Bereich von 150 bis 500 MPa. Formkörper aus Al₂O₃ haben eine Biegefestigkeit von vorzugsweise über 300 MPa, insbesondere von 500 bis 700 MPa, Formkörper aus ZrO₂ von mehr als 500 MPa insbesondere von 800 bis 1100 MPa.

Die Erfindung wird im folgenden anhand von Zeichnungen und Ausführungsbeispielen näher erläutert.

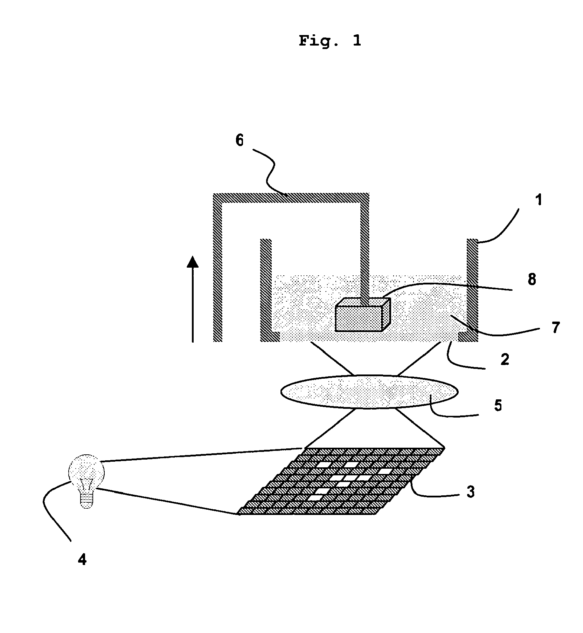

**Fig. 1** zeigt eine schematische Darstellung einer Vorrichtung zur Durchführung des erfindungsgemäßen Verfahrens. Wie Fig. 1 zu entnehmen ist, umfasst die Vorrichtung einen Behälter **1** für den erfindungsgemäßen Schlicker **7.** Der Behälter **1** wird auch als Polymerisationswanne oder Wanne bezeichnet. In der dargestellten Ausführungsform weist die Wanne **1** ein transparentes Fenster **2** auf, durch das der Schlicker von unten selektiv belichtet und ausgehärtet wird. Unterhalb der Wanne **1** ist ein computergesteuerter, beweglicher Spiegel **3,** ein sogenanntes Mikrospiegelfeld (micro-mirror array), angeordnet, das mit einer Strahlungsquelle **4** bestrahlt wird. Das Bild des Spiegels **3** wird durch eine optische Vorrichtung **5** auf das transparente Fenster **2** projiziert. Über der Wanne **1** ist ein in Z-Richtung verfahrbarer Substratträger **6** angeordnet, der den schichtweise aufgebauten Körper **8** trägt. Der Substratträger **6** kann eine hier nicht dargestellte Trägerplatte aufweisen. Der Substratträger **6** wird soweit in den Schlicker eingetaucht, dass der Abstand zwischen dem Träger **6** bzw. dem daran befestigen Körper **8** und der inneren Oberfläche der Wanne **1** der Schichtdicke der zu erzeugenden Schicht entspricht. Anschließend wird die Schlickerschicht zwischen Träger **6** und innerer Wannenoberfläche durch das transparente Fenster **2** hindurch mit Hilfe des Spiegels **3** selektiv belichtet und gehärtet. Es entstehen gehärtete Bereiche, die an dem Träger **6** anhaften. Anschließend wird der Träger **6** in Z-Richtung nach oben bewegt, so dass zwischen der anhaftenden Schicht und der inneren Wannenoberfläche wieder eine Schlickerschicht mit der gewünschten Dicke entsteht. Durch erneute Belichtung wird auch diese Schicht selektiv gehärtet und durch Wiederholung des Vorgangs der gewünschte Formkörper, vorzugsweise eine dentale Restauration, schichtweise aufgebaut.

### Ausführungsbeispiele

### Beispiel 1

### Herstellung eines ZrO₂-Schlickers und dessen stereolithographische Verarbeitung zu einem hochfesten, zahnfarbenen Keramikprüfkörper

Die in Tabelle 1 aufgeführten flüssigen Komponenten wurden vorgelegt und der Photoinitiator Irgacure 819 (Ciba) und das Praseodym-acetoacetonat darin unter Rühren gelöst. Anschliessend wurde in einem Dissolver Dispermat (Firma VMA) bei 15.000 Umdrehungen/Min das mit iso-Buttersäure oberflächenmodifizierte, 3 mol-% Y₂O₃-enthaltende ZrO₂-Pulver 3Y TZP (Firma Tosoh) portionsweise so zugeführt und für 30 Min dispergiert, bis ein hochgefüllter ZrO₂-Schlicker mit einem Füllgrad von ca. 41 Vol.-% entstand, der sich gut stereolithographisch verarbeiten ließ.

**Tabelle 1: Zusammensetzung eines stereolithographisch verarbeitbaren ZrO₂-Keramikschlickers**

| **Komponente** | **Gew.-%** |
|---|---|
| Ethoxyliertes Pentaerythritoltetraacrylat (EPTA) | 3.50 |
| Urethandiacrylat UDPA (aus TMDI und HPA) | 3.50 |
| Irgacure 819 | 0.50 |
| Hexandioldiacrylat (HDA) | 3.00 |
| Dolacol D 1003 (Zschimmer&Schwarz) | 3.90 |
| 1-Octanol | 1.50 |
| PEG-300 | 3.00 |
| ZrO₂ | 81.00 |
| Pr(III)-Acetylacetonat | 0.10 |

Der Schlicker wurde mittels der handelsüblichen Stereolithographieanlage Perfactory (Firma Envisiontec) zu zylindrischen Biaxialfestigkeits-Prüfkörpern (Durchmesser 15 mm und Höhe 2,2 mm) verbaut. Die Wärmebehandlung der Prüfkörper erfolgte in einem handelsüblichen Nabertherm Sinterofen mit katalytischer Nachverbrennung in folgenden Schritten: 1. Schritt (Entbinderung): Thermische Behandlung bis 500 °C mit einer Aufheizgeschwindigkeit von 1 K/Min. Nachfolgend eine Haltephase von 90 Min bei 500 °C. 2. Schritt (Sinterung): Erwärmung auf 1500 °C mit einer Aufheizgeschwindigkeit von 20 K/Min und einer Haltezeit von 1 h bei 1500 °C. Die Abkühlung erfolgte im Ofen innerhalb von ca. 12 h. Es wurden zahnfarbene Prüfkörper erhalten. Von den Prüfkörpern wurde nach Sinterung die Dichte nach der Auftriebsmethode bestimmt, wobei ein Wert von grösser 99 % der theoretischer Dichte erreicht wurde. Die Bestimmung der Biaxialfestigkeit erfolgte nach der ISO-Norm 6872. Es wurde ein Mittelwert der Biaxialfestigkeit von 1110 MPa (bei n = 10 Prüfkörpern) bestimmt, was dem Wert einer hochfesten Keramik entspricht.

### Beispiel 2

### Herstellung einer zahnfarbenen Krone auf der Basis des ZrO₂-Schlickers aus Beispiel 1

Auf der Basis des ZrO₂-Schlickers aus Beispiel **1** wurde in der handelüblichen Stereolithographieanlage Perfactory unter Verwendung eines STL-Datensatzes (STL = Standard Transformation Language) ein Grünling in Form einer Zahnkrone hergestellt und anschliessend analog Beispiel **1** entbindert und gesintert. Es konnte eine hochfeste zahnfarbene Zahnkrone erhalten werden.

### Beispiel 3:

### Herstellung von weiteren Keramik- oder Glaskeramikschlickern, die für eine stereolithographische Verarbeitung zur Herstellung von hochfesten, zahnfarbenen Dentalformteilen geeignet sind

Analog Beispiel 1 wurden die in Tabelle 2 aufgeführten Zusammensetzungen von Keramik- bzw. Glaskeramik-Schlickern hergestellt, die sich leicht entbindern und stereolithographisch zu hochfesten, zahnfarbenen dentalen Keramik- und Glaskeramik formteilen verarbeiten ließen.

**Tabelle 2: Zusammensetzung von stereolithographisch verarbeitbaren Keramik-und Glaskeramikschlickern zur Herstellung von Dentalformteilen**

| **Komponente** | **A** | **B** | **C** | **D** |
|---|---|---|---|---|
| | **[Gew.-%]** | **[Gew.-%]** | **[Gew.-%]** | **[Gew.-%]** |
| EPTA | 4.00 | 4.00 | 4.00 | 7.00 |
| UDPA | 5.50 | - | 3.50 | 5.50 |
| Urethandiacrylat aus TDI und HPA | - | 5.50 | - | - |
| Dibenzoyldiethylgermanium | 0.50 | - | - | - |
| Irgacure 819 | - | 0.80 | 0.50 | 0.50 |
| HDA | 3.00 | 2.50 | 3.00 | 3.00 |
| Tetramethylthiuramdisulfid | - | 0.20 | - | - |
| Byk-9077 | 2.00 | - | 1.00 | 2.00 |
| ZrO₂ (propionsäuremodifiziert) | 80.00 | 80.00 | - | - |
| Aluminiumoxid CT 300 SDT | - | - | 80.00 | - |
| GM Fräskeramik (Art 595579) | - | - | - | 75.00 |
| 1-Octanol | 1.90 | 3.90 | 4.80 | 3.80 |
| PEG-300 | 3.00 | 3.00 | 3.00 | 3.00 |
| Pr(III)-Acetylacetonat | 0.10 | - | 0.10 | 0.10 |
| Terbium-(III)-Acetylacetonat | - | 0.10 | 0.10 | 0.10 |

## Patentansprüche

1. Schlicker zur Herstellung von Keramikformteilen auf der Basis von polyreaktivem Bindemittel, Polymerisationsinitiator und Füllstoff, **dadurch gekennzeichnet, dass** er
(A) 5 - 65 Gew.-% polyreaktives Bindemittel,
(B) 0,001 - 1,0 Gew.-% Photoinitiator,
(C) 35 - 90 Gew.-% oberflächenmodifizierte Keramik-und/oder Glaskeramikpartikel
jeweils bezogen auf die Gesamtmasse des Schlickers, und Lösungsmittel enthält.

2. Schlicker nach Anspruch 1, der zusätzlich
(D) 0,001 - 1,0 Gew.-% Inhibitor enthält, bezogen auf die Gesamtmasse des Schlickers.

3. Schlicker nach Anspruch 1 oder 2, der zusätzlich (E) 0 - 20 Gew.-% Entbinderungsbeschleuniger enthält, bezogen auf die Masse der Komponente A.

4. Schlicker nach einem der Ansprüche 1 bis 3, der zusätzlich (F) 0,00001 - 2,0 Gew.-% farbgebende Komponenten enthält, bezogen auf die Masse der Komponente (C).

5. Schlicker nach einem der Ansprüche 1 bis 4, der als polyreaktives Bindemittel (A) lichthärtende radikalisch oder kationisch polymerisierbare Harze, Monomere für die ring-öffnende Metathesepolymerisation, Thiol-En-Harze oder Michael-Reaktionsharze enthält.

6. Schlicker nach einem der Ansprüche 1 bis 5, der als Komponente (C) Glaskeramikpartikel oder Keramikpartikel auf der Basis von reinem ZrO₂, reinem Al₂O₃, reinem ZrO₂-Al₂O₃, ZrO₂ oder ZrO₂-Al₂O₃, das mit HfO₂, CaO, Y₂O₃, CeO₂ und/oder MgO stabilisiert ist, enthält.

7. Schlicker nach einem der Ansprüche 1 bis 6, bei dem die Partikel der Komponente (C) mit einer linearen oder verzweigten Carbonsäure, insbesondere Ameisensäure, Essigsäure, Propionsäure, Octansäure, Isobuttersäure, Isovaleriansäure oder Pivalinsäure; einem sauren Phosphorsäureester, insbesondere Dimethyl-, Diethyl-, Dipropyl-, Dibutyl-, Dipentyl-, Dihexyl-, Dioctyl- oder Di(2-ethylhexyl)phosphat; einer Phosphonsäure, insbesondere Methyl-, Ethyl-, Propyl-, Butyl- Hexyl, Octyl- oder Phenylphosphonsäure; oder einem Silane, insbesondere Propyltrimethoxysilan, Phenyltrimethoxysilan, Hexyltrimethoxysilan, Octyltrimethoxysilan, Trimethylchlorsilan, Trimethylbromsilan, Trimethylmethoxysilan oder Hexamethyldisilazan, oberflächenmodifiziert sind, wobei das Oberflächenmodifizierungmittel keine radikalisch polymerisierbaren Gruppen aufweist.

8. Schlicker nach einem der Ansprüche 1 bis 7, bei dem die Partikel der Komponente (C) eine Partikelgröße im Bereich von 50 nm bis 50 µm aufweisen.

9. Schlicker nach einem der Ansprüche 3 bis 8, die als Entbinderungsbeschleuniger (E) einen Kettenregler, insbesondere ein Mercaptan, ein Disulfid oder einen Photoiniferter, vorzugsweise Laurylmercaptan, ein Dithiourethandisulfid, Tetramethylthiuramdisulfid oder Isopropylxanthogensäuredisulfid; ein Comonomer, das eine oder mehrere thermisch labile Gruppen, insbesondere eine oder mehrere Peroxid-, Azo- oder Urethangruppen aufweist; ein Comonomer mit einer ceiling-Temperatur von -10 bis 150 °C, insbesondere α-Methylstyrol oder Polytetrahydrofuran (PTHF); oder ein Telechel mit radikalisch polymerisierbaren Gruppen enthält, insbesondere ein PTHF-Di(meth)acrylat-Telechel.

10. Schlicker nach einem der Ansprüche 4 bis 9, der als farbgebende Komponente (F) eine Übergangsmetallverbindung enthält insbesondere ein Acetylacetonat oder ein Carbonsäuresalz der Elemente Eisen, Cer, Praseodym, Terbium, Lanthan, Wolfram, Osmium, Terbium und Mangan, insbesondere Eisen(III)-acetat oder Eisen(III)-acetylacetonat, Mangan(III)-acetat oder Mangan(III)-acetylacetonat, Praseodym(III)-acetat oder Praseodym(III)-acetylacetonat oder Terbium(III)-acetat oder Terbium(III)-acetylacetonat.

11. Schlicker nach einem der Ansprüche 1 bis 10, der zusätzlich ein Lösungsmittel enthält, das aus Phthalaten, insbesondere Dibutyl- oder Dihexylphthalat, nichtsauren Phosphaten, insbesondere Tributyl- oder Trikresylphosphat, n-Octanol, Glycerin, Polyethylenglykolen, Octanol, Triethylenglycol-Divinylether, 2-Amino-2-methyl-1-propanol, 2-Methyl-2,4-pentandiol, Ammoniumcitrat tribasisch (fest), Tripropylenglycol, Tetraethylenglycol, Triethylenglycol, Triethylcitrat, Acet-Essigsäure-Ethylester, Cyclohexanol, Cyclohexanon, Diethylenglycolmonomethylether, Oxalsäuredibutylester, 2,5-Dimethoxyterahydrofuran, Polyethylenglycol 300, 1- oder 2-Nonanol, Diethylenglycoldiethylether, 2,5-Dimethoxytetrahydrofuran, Oxalsäuredibutylester, Cyclohexanol, Cyclohexanon, Acetessigsäureethylester und Mischungen davon ausgewählt ist.

12. Schlicker nach einem der Ansprüche 1 bis 11, der eine Viskosität im Bereich von 200-20000 Pas, bevorzugt 500-5000 Pas aufweist.

13. Verwendung eines Schlickers gemäß einem der Ansprüche 1 bis 12 zur Herstellung von Keramik- oder Glaskeramikformteilen.

14. Verwendung nach Anspruch 13, wobei das Keramikformteil eine Dentalrestauration ist, wie z.B. ein Inlay, Onlay, Veneer, eine Krone, Brücke oder ein Gerüst.

15. Verfahren zur Herstellung eines Keramik- oder Glaskeramikformteils bei dem man
(a) einen Grünling herstellt, indem man einen Schlicker gemäß einem der Ansprüche 1 bis 10 durch lokales Einbringen von Strahlungsenergie unter Ausbildung der geometrischen Form des Grünlings aushärtet,
(b) man dann den Grünling einer Wärmebehanldung zur Entfernung des Bindemittels (Entbinderung) unterwirft, um einen Weissling zu erhalten, und
(c) man den Weissling sintert.

## Claims

1. Slip for manufacturing ceramic mouldings based on a polyreactive binder, polymerisation initiator and filler, **characterised in that** it comprises
(A) 5 - 65 wt % polyreactive binder,
(B) 0.001 - 1.0 wt % photoinitiator,
(C) 35 - 90 wt % surface-modified ceramic and/or glass ceramic particles, in each case relative to the overall mass of the slip, and solvent.

2. Slip according to claim 1, additionally comprising
(D) 0.001 - 1.0 wt % inhibitor, relative to the overall mass of the slip.

3. Slip according to claim 1 or 2, additionally comprising
(E) 0 - 20 wt % debinding accelerator, relative to the mass of component A.

4. Slip according to one of claims 1 to 3, additionally comprising (F) 0.00001 - 2.0 wt % chromophoric components, relative to the mass of component (C).

5. Slip according to one of claims 1 to 4 comprising light-curing radically or cationically polymerisable resins, monomers for ring-opening metathesis polymerisation, thiol-ene resins or Michael reaction resins as the polyreactive binder (A).

6. Slip according to one of claims 1 to 5 comprising glass ceramic particles or ceramic particles based on pure ZrO₂, pure Al₂O₃, pure ZrO₂-Al₂O₃, ZrO₂ or ZrO₂-Al₂O₃, which is stabilised with HfO₂, CaO, Y₂O₃ CeO₂ and/or MgO, as the component (C).

7. Slip according to one of claims 1 to 6, in which the particles of component (C) are surface-modified with a linear or branched carboxylic acid, in particular formic acid, acetic acid, propionic acid, octanoic acid, isobutyric acid, isovaleric acid or pivalic acid; an acidic phosphoric acid ester, in particular dimethyl, diethyl, dipropyl, dibutyl, dipentyl, dihexyl, dioctyl or di(2-ethylhexyl) phosphate; a phosphonic acid, in particular methyl, ethyl, propyl, butyl, hexyl, octyl or phenylphosphonic acid; or a silane, in particular propyltrimethoxysilane, phenyltrimethoxysilane, hexyltrimethoxysilane, octyltrimethoxysilane, trimethylchlorosilane, trimethylbromosilane, trimethylmethoxysilane or hexamethyldisilazane, wherein the surface-modification agent does not possess a radically polymerisable group.

8. Slip according to one of claims 1 to 7, in which the particles of component (C) have a particle size in the range from 50 nm to 50 µm.

9. Slip according to one of claims 3 to 8 comprising as the debinding accelerator (E) a chain transfer agent, in particular a mercaptan, a disulfide or a photoiniferter, preferably lauryl mercaptan, a dithiourethane disulfide, tetramethylthiuram disulfide or isopropylxanthogenic acid disulfide; a comonomer that possesses one or more thermally labile groups, in particular one or more peroxide, azo or urethane groups; a comonomer with a ceiling temperature from -10 to 150 °C, in particular α-methylstyrene or polytetrahydrofuran (PTHF); or a telechel with radically polymerisable groups, in particular a PTHF di(meth)acrylate telechel.

10. Slip according to one of claims 4 to 9 comprising as the chromophoric component (F) a transition metal compound, in particular an acetyl acetonate or a carboxylic acid salt of the elements iron, cerium, praseodymium, terbium, lanthanum, tungsten, osmium, terbium and manganese, in particular iron (III) acetate or iron (III) acetyl acetonate, manganese (III) acetate or manganese (III) acetyl acetonate, praseodymium (III) acetate or praseodymium (III) acetyl acetonate or terbium (III) acetate or terbium (III) acetyl acetonate.

11. Slip according to one of claims 1 to 10 additionally comprising a solvent that is selected from phthalates, in particular dibutyl or dihexyl phthalate, non-acidic phosphates, in particular tributyl or tricresyl phosphate, n-octanol, glycerol, polyethylene glycols, octanol, triethylene glycol divinyl ether, 2-amino-2-methyl-1-propanol, 2-methyl-2,4-pentane diol, tribasic ammonium citrate (solid), tripropylene glycol, tetraethylene glycol, triethylene glycol, triethyl citrate, ethyl acetoacetate, cyclohexanol, cyclohexanone, diethylene glycol monomethyl ether, dibutyl oxalate, 2,5-dimethoxytetrahydrofuran, polyethylene glycol 300, 1- or 2-nonanol, diethylene glycol diethyl ether, 2,5-dimethoxytetrahydrofuran, dibutyl oxalate, cyclohexanol, cyclohexanone, ethyl acetoacetate and mixtures thereof.

12. Slip according to one of claims 1 to 11 which has a viscosity in the range of 200-20000 Pas, preferably 500-5000 Pas.

13. Use of a slip according to one of claims 1 to 12 for the manufacture of ceramic or glass ceramic mouldings.

14. Use according to claim 13, wherein the ceramic moulding is a dental restoration, such as e.g. an inlay, onlay, veneer, a crown, bridge or a framework.

15. Process for the manufacture of a ceramic or glass ceramic moulding, in which process
(a) a green body is manufactured by curing a slip according to one of claims 1 to 10 by locally introducing radiation energy to form the geometric shape of the green body,
(b) the green body is then subjected to a heat treatment to remove the binder (debinding), in order to obtain a white body, and
(c) the white body is sintered.

## Revendications

1. Barbotine destinée à la fabrication de pièces façonnées en céramique, à base d'un liant polyréactif, d'un amorceur de polymérisation et d'une charge, **caractérisée en ce qu'**elle contient :
A) de 5 à 65 % en poids de liant polyréactif,
B) de 0,001 à 1,0 % en poids de photo-amorceur,
C) et de 35 à 90 % en poids de particules de céramique et/ou de vitrocéramique, modifiées en surface, dans chaque cas par rapport à la masse totale de la barbotine,
et un solvant.

2. Barbotine conforme à la revendication 1, qui contient en outre :
D) de 0,001 à 1,0 % en poids d'inhibiteur,
par rapport à la masse totale de la barbotine.

3. Barbotine conforme à la revendication 1 ou 2, qui contient en outre :
E) de 0 à 20 % en poids d'accélérateur de déliantage,
par rapport à la masse de composant (A).

4. Barbotine conforme à l'une des revendications 1 à 3, qui contient en outre :
F) de 0,00001 à 2,0 % en poids de composants colorants,
par rapport à la masse de composant (C).

5. Barbotine conforme à l'une des revendications 1 à 4, qui contient, en tant que liant polyréactif (A), des résines photodurcissables, polymérisables par voie radicalaire ou cationique, des monomères pour polymérisation par métathèse avec ouverture de cycle, des résines de type thiol-ène, ou des résines de réaction de Michaël.

6. Barbotine conforme à l'une des revendications 1 à 5, qui contient, en tant que composant (C), des particules de vitrocéramique ou des particules de céramique, à base de zircone ZrO₂ pure, d'alumine Al₂O₃ pure, de zircone-alumine pure, ou de zircone ou zircone-alumine stabilisée avec de l'oxyde de hafnium HfO₂, de l'oxyde de calcium CaO, de l'oxyde d'yttrium Y₂O₃, de l'oxyde de cérium CeO₂ et/ou de l'oxyde de magnésium MgO.

7. Barbotine conforme à l'une des revendications 1 à 6, dans laquelle les particules de composant (C) sont modifiées en surface avec un acide carboxylique linéaire ou ramifié, en particulier de l'acide formique, de l'acide acétique, de l'acide propionique, de l'acide octanoïque, de l'acide isobutyrique, de l'acide isovalérique ou de l'acide pivalique, avec un ester phosphate acide, en particulier du phosphate de diméthyle, de diéthyle, de dipropyle, de dibutyle, de dipentyle, de dihexyle, de dioctyle ou de di(2-éthyl-hexyle), avec un acide phosphonique, en particulier de l'acide méthyl-phosphonique, éthyl-phosphonique, propyl-phosphonique, butyl-phosphonique, hexyl-phosphonique, octyl-phosphonique ou phényl-phosphonique, ou avec un silane, en particulier du propyl-triméthoxy-silane, du phényl-triméthoxy-silane, de l'hexyl-triméthoxy-silane, de l'octyl-triméthoxy-silane, du triméthylchloro-silane, du triméthyl-bromo-silane, du triméthyl-méthoxy-silane ou de l'hexaméthyl-disilazane, étant entendu que l'agent de modification de surface ne comporte aucun groupe qui puisse donner lieu à une polymérisation par voie radicalaire.

8. Barbotine conforme à l'une des revendications 1 à 7, dans laquelle les particules de composant (C) présentent une taille de particules située dans la gamme allant de 50 nm à 50 µm.

9. Barbotine conforme à l'une des revendications 3 à 8, qui contient, en tant qu'accélérateur de déliantage (E), un agent de régulation des chaînes, en particulier un thiol, un disulfure ou un agent de photoamorçage-transfert-terminaison (agent "photoiniferter"), de préférence du lauryl-mercaptan, un disulfure de dithiouréthane, du disulfure de tétraméthyl-thiurame ou du disulfure d'acide isopropyl-xanthogénique, un comonomère comportant un ou plusieurs groupe(s) thermolabile(s), en particulier un ou plusieurs groupes peroxy, azo ou uréthane, un comonomère dont la température plafond se situe entre -10 et 150 °C, en particulier de l'alpha-méthyl-styrène ou du polytétrahydrofurane, ou un composé téléchélique comportant des groupes pouvant donner lieu à une polymérisation par voie radicalaire, en particulier un composé téléchélique bis(acrylate) ou bis(méthacrylate) de polytétrahydrofurane.

10. Barbotine conforme à l'une des revendications 4 à 9, qui contient, en tant que composant colorant (F), un composé de métal de transition, en particulier un acétyl-acétonate ou un carbonate de l'un des éléments fer, cérium, praséodyme, terbium, lanthane, tungstène, osmium et manganèse, en particulier de l'acétate de fer-III, de l'acétyl-acétonate de fer-III, de l'acétate de manganèse-III, de l'acétyl-acétonate de manganèse-III, de l'acétate de praséodyme-III, de l'acétyl-acétonate de praséodyme-III, de l'acétate de terbium-III ou de l'acétyl-acétonate de terbium-III.

11. Barbotine conforme à l'une des revendications 1 à 10, qui contient en outre un solvant qui est choisi parmi les suivants : phtalates, en particulier du phtalate de dibutyle ou du phtalate de dihexyle, phosphates non acides, en particulier du phosphate de tributyle ou du phosphate de tricrésyle, n-octanol, glycérol, polyéthylèneglycols, octanol, éther divinylique de triéthylèneglycol, 2-amino-2-méthyl-propane-1-ol, 2-méthyl-pentane-2,4-diol, citrate d'ammonium tribasique (solide), tripropylèneglycol, tétraéthylèneglycol, triéthylèneglycol, citrate de triéthyle, acétyl-acétate d'éthyle, cyclohexanol, cyclohexanone, éther monométhylique de diéthylèneglycol, oxalate de dibutyle, 2,5-diméthoxy-tétrahydrofurane, polyéthylèneglycol 300, nonane-1-ol, nonane-2-ol, et éther diéthylique de diéthylèneglycol, en particulier 2,5-diméthoxy-tétrahydrofurane, oxalate de dibutyle, cyclohexanol, cyclohexanone et acétyl-acétate d'éthyle, ainsi que leurs mélanges.

12. Barbotine conforme à l'une des revendications 1 à 11, qui présente une viscosité située dans l'intervalle allant de 200 à 20 000 Pa.s, et de préférence, de 500 à 5000 Pa.s.

13. Utilisation d'une barbotine conforme à l'une des revendications 1 à 12 pour la fabrication de pièces façonnées en céramique ou en vitrocéramique.

14. Utilisation conforme à la revendication 13, dans laquelle la pièce en céramique est une pièce de restauration dentaire, comme par exemple une incrustation "inlay" ou "onlay", une facette "veneer", une couronne, un bridge ou une armature.

15. Procédé de fabrication d'une pièce façonnée en céramique ou en vitrocéramique, dans lequel :
a) on prépare un "corps vert" (ébauche crue), en faisant durcir une barbotine conforme à l'une des revendications 1 à 10 par application locale d'un rayonnement énergétique, tout en donnant au corps vert une forme d'une certaine géométrie ;
b) on soumet ce corps vert à un traitement thermique pour en chasser le liant (déliantage), ce qui donne un "corps blanc" (pièce non frittée),
c) et l'on fritte ce corps blanc.
